(19) **Europäisches Patentamt**

**European Patent Office**

**Office européen des brevets**

(11) **EP 0 602 686 B1**

(12) **EUROPÄISCHE PATENTSCHRIFT**

(45) Veröffentlichungstag und Bekanntmachung des Hinweises auf die Patenterteilung:
**12.09.2001   Patentblatt 2001/37**

(51) Int Cl.$^7$: **A61K 35/78**, A61K 45/00

(21) Anmeldenummer: **93120461.4**

(22) Anmeldetag: **17.12.1993**

(54) **Lektinkonzentrate aus Mistelextrakten und entsprechende standardisierte, stabilisierte Mistellektinpräparate, Verfahren zu ihrer Herstellung sowie diese enthaltende Arzneimittel und deren Verwendung zur Erhöhung der natürlichen Immunresistenz und/oder in der Tumor-Therapie**

Lectin concentrates of mistletoe extracts and corresponding standardized, stabilized mistletoe lectin preparations, process for their production as well as medicines containing them and their use

Concentrés des lectines d'extraits de gui et compositions standardisées, stabilisées des lectines de gui correspondantes, procédé pour leur préparation, ainsi que médicaments les contenant et leur utilisation

(84) Benannte Vertragsstaaten:
**AT BE CH DE DK ES FR GB GR IE IT LI LU MC NL PT SE**

(30) Priorität: **18.12.1992   DE 4242902**

(43) Veröffentlichungstag der Anmeldung:
**22.06.1994   Patentblatt 1994/25**

(73) Patentinhaber: **MADAUS Aktiengesellschaft D-51109 Köln (DE)**

(72) Erfinder:
• **Dr. Hajto, Tibor**
  **Ch-4104 Oberwil (CH)**
• **Hostanska, Katarina, Dr. Chem.-Ing.**
  **CH-4142 Münchenstein (CH)**

(74) Vertreter: **Wolff, Felix, Dr. et al Kutzenberger & Wolff Theodor-Heuss-Ring 23 50668 Köln (DE)**

(56) Entgegenhaltungen:
**DE-A- 3 801 391          DE-U- 9 208 913**

• CANCER RESEARCH, Bd.49, Nr.17, 1. September 1989, USA Seiten 4803 - 4808 T. HAJTO ET AL 'Modulatory Potency of the beta-Galactoside-specific Lectin from Mistletoe Extract (Iscador) on the Host Defence System in Vivo in Rabbits and Patients'
• CANCER RESEARCH, Bd.50, Nr.10, 15. Mai 1990, USA Seiten 3322 - 3326 T. HAJTO ET AL 'Increased Secretion of Tumor Necrosis Factor alpha, Interleukin 1, and Interleukin 6 by Human Mononuclear Cells exposed to beta-Galactoside-specific Lectin from Clinically Applied Mistletoe Extract'
• DATABASE WPI Week 8636, Derwent Publications Ltd., London, GB; AN 86-232840 & DD-A-235 418 (STAATLICHES INST IM) 7. Mai 1986

EP 0 602 686 B1

**Beschreibung**

Die Erfindung betrifft Mistellektin-Konzentrate mit hohem Anteil an immunologisch aktiven galaktosidspezifischen Mistellektinen aus wässrigen Mistelextrakten und entsprechende standardisierte, stabilisierte immunologisch aktive Mistellektin-Fraktionen enthaltenden Präparate, ferner ein Verfahren zu ihrer Herstellung, wobei man wässrige Mistelextrakte einer fraktionierten Filtration unterwirft und die Endprodukte standardisiert und stabilisiert sowie Arzneimittel enthaltend standardisierte, stabilisierte Präparate mit definiertem Gehalt an immunologisch aktiven Mistellektin-Fraktionen sowie die Verwendung dieser Mistellektine zur Herstellung eines Arzneimittels zur Erhöhung der natürlichen Immunresistenz bei Menschen und Säugetieren und/oder in der Tumor-Therapie.

Die Mistel (Viscum album) ist seit dem Altertum als Heil- und Zaubermittel bekannt; deren Extrakte sowie Herba visci enthaltende Teemischungen sind auch heute noch als blutdrucksenkende, Cholin-ähnlich wirkende Mittel sowie als Mittel zur Behandlung von Arthrosen und rheumatischen Erkrankungen im Gebrauch. Ausserdem ist die Verwendung von Mistelextrakten als krebshemmende Mittel in der **DD-A1-235 418** beschrieben worden. Wirksamer Bestandteil sind dabei als Lektine bezeichnete Inhaltsstoffe, bei denen es sich um Eiweissstoffe handelt, die sehr spezifisch Saccharide und Polysaccharide, auch in Lipid- oder Protein-gebundener Form zu erkennen und zu binden vermögen. Die Spezifität der jeweils in Betracht kommenden Lektine hängt hauptsächlich von ihrer Abstammung ab und ist für ihre Verwendung in der Serodiagnostik und anderen Methoden der Biochemie und der Molekularbiologie von Bedeutung.

Mistelpflanzen und deren Extrakte enthalten drei Arten von toxischen Proteinen mit einer Spezifität für den Kohlenwasserstoffanteil der Glucokonjugate, und zwar die Lektine ML I, ML II und ML III [Franz, H., Ziska, P., Kindt, A. **Biochem. J.**, **195** (1981) 481 bis 484]. Die Toxizität der Mistelextrakte wurde, zumindest anfänglich, der Anwesenheit eines galaktosidspezifischen Lektins (ML I) zugeschrieben [Holtskog, R., Sandvig, K., Olsnes, S. **Oncology, 45** (1988) 172 bis 179]. Die zytotoxische Wirksamkeit von ML I wurde unter Verwendung von Kulturen von menschlichen peripheren mononucleären Blutzellen [PBMC] unter Anwendung von drei, von einander unabhängigen Methoden untersucht. Dabei wurde gefunden, dass die Lebensfähigkeit von Zellen durch Lektinkonzentrationen $\geq$ 10 ng/kg in signifikanter Weise beeinträchtigt wird. Dieser Wert für die Lektinkonzentration entspricht nur seinem biologisch aktiven, für die Bindung von Zucker verantwortlichen Teil, welcher mittels eines optimierten Enzymtests [Hajto, T., Hostanska, K., Gabius, H.-J. **Cancer Res., 49** (1989) 4803 bis 4808] bestimmt wurde. Bei Verabreichung in kleineren, nicht toxischen Dosierungen zeigte ML I augenscheinlich eine leicht mitogene Wirkung und induzierte so eine Zunahme der Zytotoxizität natürlicher Killerzellen [NK] gegenüber $K_{562}$-Zielzellen. Eine ähnliche NK-Zunahme wurde auch beobachtet, wenn für das Immunsystem nicht-toxische Lektin-Dosen verabreicht wurden. Die optimierte Anwendung von zuckerbindendem, aktivem Lektin in Mistelextrakten, bestimmt mittels ELLA, verstärkte verschiedene Abwehr-Mechanismen des Empfängers und ermöglichte eine Verminderung des Tumorwachstums bei Patienten.

Die DE-U 92 08 913 beschreibt aus wäßrigen Mistel-Extrakten aufgereinigtes Mistellektin I. Dieses Mistellektin wird unter Zuhilfenahme von affinitätschromatographischen Methoden angereichert, um zu versuchen, den immunmodulatorischen Effekt quantitativ erfaßbar zu machen.

Die Zytotoxische Wirkung von Mistelextrakten wurde, wie bereits erwähnt, hauptsächlich der Anwesenheit von ML 1 zugeschrieben. Zu einem geringeren Teil tragen andere Arten von Lektinen (ML II und ML III) sowie eine kleine Gruppe von stark basischen Proteinen mit einem Molekulargewicht von annähernd 5 kd, und zwar Viscotoxin $A_2$, $A_3$ und B zur zytotoxischen Wirkung von Mistelextrakten bei. ML I ist für D-Galaktose und ML III für N-Acetylaminogalaktosamin spezifisch, während ML II sowohl für D-Galaktose als auch für N-Acetylaminogalaktosamin spezifisch ist. Die hohe Toxizität von Mistellektinen wurde ebenfalls bestätigt. Die $LD_{50}$-Werte liegen im Bereich von 0,001 bis 0,0001 g/kg [Franz, H. **Oncology, 43,** Supplement 1, (1986) 23 bis 34 und Ziska, P., Gelbin, M., Franz, H. **Lectins, Vol. 8** (1991) im Druck]. Folglich kann die Anwendung höherer, toxischer und nicht optimierter Dosierungen von Mistellektinen eine Suppressions-Wirkung auf verschiedene Abwehr-Mechanismen des Empfängers auslösen. Erfolgt jedoch die Anwendung in einer kleineren, für das Immunsystem nicht-toxischen Dosierung, ähnlich wie bei anderen Lektinen, so ist die Möglichkeit gegeben, dass Mistellektine in der Lage sind, eine immunmodulierende Antwort hervorzurufen [Franz, H. **Oncology, 43,** Supplement 1 (l.c.) und Metzner, G., Franz, H., Kindt, A. Fahlbusch, B., Süss, J. **Immunbiol., 169** (1985) 461 bis 471]. In der Tat sind in dieser Weise optimierte Dosierung von gereinigtem ML I befähigt, verschiedene Parameter des Abwehrsystems des Empfängers in vivo zu stimulieren [Hajto, T., Hostanska, K., Gabius, H.-J. **Cancer Res. 49,** (1989) 4803 bis 4808], was zumindest zum Teil durch eine Lektin-induzierte vermehrte Sekretion von Tumor-Nekrose-Faktor-alpha [TNF-alpha], Interleukin-1 [IL-1] und Interleucin-6 [IL-6] vermittelt wird [Hajto, T., Hostanska, K., Frei, K., Rordorf, C., Gabius, H.-J. **Cancer Res., 50** (1990) 3322 bis 3326]. Die Anwesenheit des spezifischen Zuckers (D-Galaktose) verminderte die Lektin-induzierte Freisetzung von Zytokinen aus menschlichen peripheren mononucleären Blutzellen [PBMC] und bestätigte die Mitwirkung der Protein-Zucker-Wechselwirkung an diesen Immunantworten: Hajto, T., Hostanska, K., Frei, K., Rordorf, C., Gabius, H.-J. [**Cancer Res., 50** (l.c.)].

Aufgrund der starken Toxizität der Mistelextrakte bzw. der darin enthaltenen Lektine, insbesondere von ML I sowie aufgrund der Tatsache, dass die biologische Aktivität und die Wirkung auf das Immunsystem und damit die Verträglichkeit von Mistelextrakten und deren Inhaltstoffen in hohem Masse von deren Dosierung, insbesondere deren exakten und zuverlässigen Einstellung abhängt, ist es für Therapie und Prophylaxe von ausschlaggebender Bedeutung, dass Konzentrate von- Mistelextrakten mit einem hohen Anteil an immunologisch aktiven galaktosidspezifischen Mistellektinen und genau bekannter Zusammensetzung, vorallem mit genau bekannter Konzentration der wirksamen Inhaltsstoffe verfügbar gemacht und standardisierte Präparate einem genau definierten Gehalt an ausgewählten immunologisch aktiven Mistellektin-Fraktionen zur Verfügung gestellt werden. Da sich gezeigt hat, dass der Einsatz von Mistellektinen auf dem Gebiet der Tumor-Therapie erfolgversprechend ist, entstand eine starke Nachfrage nach hierfür geeigneten Mistellektin-Fraktionen und diese enthaltenden Präparaten.

Aufgabe der Erfindung ist es daher, (a) Mistellektin-Konzentrate mit hohem Anteil an immunologisch aktiven galaktosidspezifischen Mistellektinen und entsprechende standardisierte, stabilisierte Mistellektinpräparate für die Verwendung in der Tumor-Therapie und zur Erhöhung der natürlichen Immunresistenz, welche für die jeweilige therapeutische oder prophylaktische Verwendung notwendige eine exakte Dosierung ermöglichen, sowie (b) ein Verfahren zur Herstellung der Mistellektin-Konzentrate mit hohem Anteil an immunologisch aktiven galaktosidspezifischen Mistellektinen sowie (c) ein Verfahren zur Herstellung der entsprechenden standardisierten Mistellektinpräparate.

Die gestellte Aufgabe wird gelöst, indem man

a) Lektinkonzentrate aus Mistelextrakten und entsprechende standardisierte Mistellektinpräparate mit einem definierten hohen Gehalt an immunologisch aktiven galaktosidspezifischen Mistellektinen bestimmt als Mistellektin-I-Standard, wobei sich die Lektinmenge nur auf die biologisch aktiven Lektine bezieht, zur Verfügung stellt,

b) zur Herstellung von Mistellektin-Konzentraten mit hohem Anteil an immunologisch aktiven galaktosidspezifischen Mistellektinen wässrige Mistelextrakte einer fraktionierten Filtration unter Verwendung unterschiedlicher Filter und Waschflüssigkeiten unterwirft, wobei man zwei Fraktionierungsstufen durchführt, wobei man in der ersten Fraktionierungsstufe einen unfermentierten wässrigen Mistelextrakt mindestens einer Ultrafiltration zur Gewinnung einer Fraktion mit Substanzen mit Molekularmassen von 20'000 bis 100'000 unterwirft, das durch Ultrafiltrationen erhaltene Konzentrat in der zweiten Fraktionierungsstufe mindestens einer Sterilfiltration unterwirft und das dabei erhaltene Endkonzentrat gegebenenfalls auf das gewünschte Endvolumen verdünnt und gegebenenfalls durch geeignete Stabilisatoren stabilisiert; und

c) zur Herstellung von standardisierten, immunologisch aktive Mistellektinfraktionen enthaltende Präparaten die biologische Aktivität der nach dem unter (a) beschriebenen Verfahren erhaltenen Mistellektin-Konzentrate bestimmt, die Konzentration der immunologisch aktiven galaktosidspezifischen Mistellektine in der erhaltenen Fraktion bestimmt, die Fraktion mit einer physiologisch verträglichen Pufferlösung verdünnt, gegebenenfalls durch geeignete Stabilisatoren stabilisiert und auf den für die vorgesehene therapeutische Verwendung erforderlichen Lektingehalt einstellt, wobei der Lektingehalt in biologischen Einheiten angegeben wird, proportioniert, wobei jede Teilmenge die für die vorgesehene Verwendung in Therapie und/oder Prophylaxe erforderliche Dosismenge an biologisch aktiven galaktosidspezifischen Mistellektinen enthält, und die Teilmenge einzeln unter sterilen Bedingungen in sterile und pyrogenfreie Ampullen einschließt oder gegebenenfalls lyophilisiert.

Eine Ausführung der unter (a) beschriebenen Produkte sind die Lektinkonzentrate aus Mistelextrakten und entsprechende standardisierte, stabilisierte Mistellektinpräparate in Form einer Lösung mit einem Gehalt von 50 ng/ml bis 350 ng/ml an immunologisch aktiven, galaktosidspezifischen Mistellektinen und mit einem pH-Wert von 7,o bis 7,5 und eine andere Ausführung ist eine entsprechende lyophilisierte Form.

Bei der Durchführung des unter (b) beschriebenen Verfahrens geht man vorteilhafterweise so vor, daß man in der ersten Fraktionierungsstufe zwei Ultrafiltrationen durchführt, wobei man für die erste Ultrafiltration ein steril mit bidestilliertem Wasser gewaschenes Polysulfonfilter mit 100'000 NMGT verwendet und die Filtration ohne Anwendung von Druck durchführt und anschließend das erhaltene Filtrat zweimal mit einer Pufferlösung wäscht, und daß man für die zweite Ultrafiltration ein steril mit bidestilliertem Wasser gewaschenes Celluloseacetatfilter mit 20'000 NMGT verwendet und die Filtration ohne Anwendung von Druck durchführt.

Nach der ersten Fraktionierungsstufe führt man zweckmäßigerweise eine Sterilfiltration unter Verwendung eines Filters mit einer Selektionsgrenze 0,2 μm bei maximal 600 kPa als zweite Fraktionierungsstufe durch. In der zweiten Fraktionierungsstufe werden bevorzugt zwei Sterilfiltrationen durchgeführt.

In der Regel arbeitet man im Bereich von pH-Wert 7,0 bis 7,5.

Bevorzugt führt man die erste und die zweite Fraktionierungsstufe unmittelbar aufeinanderfolgend innerhalb von 2 Stunden durch.

Das am Ende der zwei Fraktionierungsstufen erhaltene Endkonzentrat wird vorteilhafterweise auf ein vorgegebenes Volumen verdünnt, ggf. durch geeignete Stabilisatoren stabilisiert und zur Aufbewahrung in sterile, pyrogenfreie Ampullen eingeschlossen oder gegebenenfalls lyophilisiert.

Zur Stabilisierung der bereits verdünnten Lösung werden Stabilisatoren zugesetzt. Diese können Alkanole speziell Polyole und deren Derivate, wie z.B. Kohlenhydrate wie Glucose, Saccharose, Cyclodextrine, derivatisierte Cyclodextrine, Pentaerythrit, Mannit, Sorbit und Polyhydroxyfettsäuren wie Polymilchsäure und Polymilchsäureester, als auch andere hydrophile Polymere wie Polyvinylpyrrolidon, Polyvinylalkohol, als auch Aminosäuren oder Proteine wie Glycin oder Albumin spez. Humanserumalbumin sein.

Bei der Durchführung des unter (c) beschriebenen Verfahrens geht man zweckmäßigerweise so vor, daß man die Bestimmung der biologischen Aktivität mit Hilfe von drei unabhängigen Testmethoden in vivo durchgeführt, wobei man den Anteil an zuckerbindenden aktiven Lektinen unter Anwendung eines optimierten Enzym-Tests bestimmt, die Zellvitalität in einer Kultur von menschlichen peripheren mononukleären Zellen [PMNC] mittels Trypanblau und Fluoresceindiacetat, welches zu Fluorescein hydrolysiert wird, ermittelt und bei lektinempfindlichen Leukämiezellen [K 562] die Aufnahme von [$^3$H]-Thymidin bestimmt.

Die Stabilisierung kann man alternativ bei (c) nach der Verdünnung mit einer physiologisch verträglichen Pufferlösung und vor der Einstellung auf den für die vorgesehene therapeutische Verwendung erforderlichen Lektingehalt vornehmen.

Die Standardisierung der erhaltenen, biologisch aktiven galaktosidspezifischen Mistellektinfraktionen enthaltenden Präparate wird vorteilhafterweise anhand der aus der Bestimmung der biologischen Aktivität erhaltenen Werte vorgenommen.

Bei der Standardisierung ist festzustellen, daß die bestimmten Einheiten (1 ng) eine biologisch aktive Lektinmenge bedeutet und nicht anwesender Mistellektin-I-Standard-Proteingehalt.

Die biologische Aktivität wird u.a. mit nachfolgenden Untersuchungen gemessen:

1) Der Gehalt an zuckerbindenden Lektinen wird durch ein Enzymtest ELLA (Enzym-Linked-Lektin-Assay) bestimmt.

2) Messung der zytotoxischen Wirkung auf Humanlymphozyten.

3) Messung der Wirkung auf NK-Zellen in vivo.

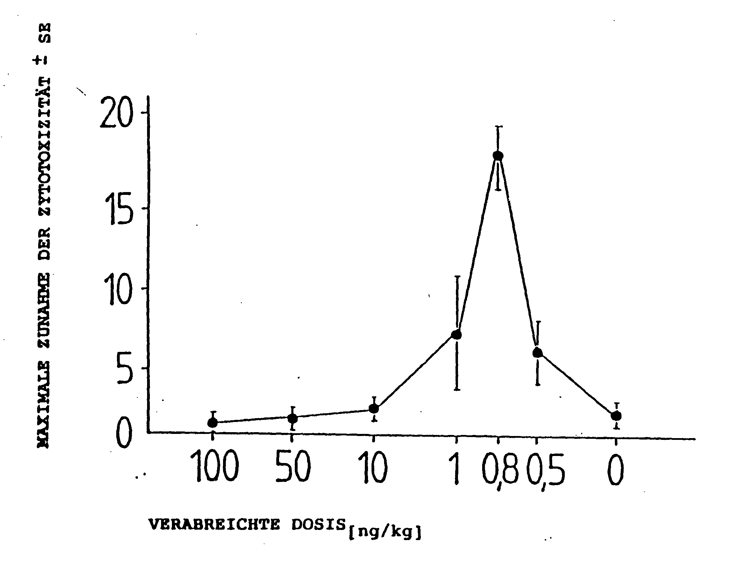

Die Erfindung wird nachfolgend anhand der beispielhaften Bechreibung der Herstellungsverfahren und der Beschreibung der Versuche zur Bestimmung der biologischen bzw. immunologischen Aktivität sowie anhand der Zeichnung erläutert. Diese zeigt die maximale Zunahme der lektin-induzierten zytotoxischen Aktivität von NK-Zellen in vivo nach 24 bis 72 Stunden nach einmaliger Injektion von ML I, in schaubildlicher Darstellung.

## BESCHREIBUNG DER HERSTELLUNGSVERFAHREN

### Herstellung der Mistellektin-Konzentrat

Für die Durchführung des Verfahrens wird zweckmäßigerweise ein Sartocon-Mini-Crossflow-System von Sartorius GmbH verwendet. Dieses ist ein modulares Ultra- und Mikrofiltrationssystem für die Aufkonzentrierung und Abtrennung von Proteinen und einer Reihe von anderen Substanzen. Dabei wird ein wäßriger Mistelektrakt, wie er durch Extraktion von frischen oder getrockneten Mistelblättern mit Wasser oder im Falle von frischen Blättern, mit einer wässrigen Pufferlösung (PBS 1%) gewonnen wird, mittels einer Schlauchquetschpumpe mit einer Förderleistung von 100 bis 900 Liter/Stunde aus einem Vorlagegefäss in das System eingebracht und durch die einzelnen Filterstufen gefördert. Das nach dem Passieren von einem oder mehreren Filtern erhaltene Filtrat wird entweder in einem Sammelgefäss aufgefangen oder zum weiteren Konzentrieren in das Vorlagegefäss zurückgeleitet und von dort erneut in das System eingebracht. Der in das System eingebrachte wässrige Extrakt, der vorteilhafterweise auf pH 7 eingestellt ist, wird zunächst einer ersten Ultrafiltration unter Verwendung eines steril mit bidestilliertem Wasser gewaschenen Polysulfonfilter mit 100 000 NMGT unterworfen. Diese Ultrafiltration wird zweckmässigerweise ohne Anwendung von Druck durchgeführt. Das erhaltene Filtrat wird vorzugsweise einer zweiten Ultrafiltration unter Verwendung eines ebenfalls steril mit bidestilliertem Wasser gewaschenen Celluloseacetatfilters mit 20 000 NMGT unterworfen, wobei diese Ultrafiltration ohne Druckanwendung durchgeführt wird. Das dabei erhaltene Konzentrat wird gesammelt, gegebenenfalls zwischenzeitlich unter sterilen Bedingungen in Ampullen eingeschlossen, und mindestens einer Sterilfiltration unterworfen. Die Sterilfiltration wird zweckmässigerweise unter Verwendung eines Membranfilters mit einer Selektionsgrenze bei 0,2 μm durchgeführt.

### Herstellung der standardisierten Präparate

Der nach der Sterilfiltration erhaltene Extrakt wird nach der Bestimmung seines Gehaltes an biologisch aktivem Lektin oder biologisch aktiven Lektinen in der weiter unten beschriebenen Weise auf die optimale Verdünnung eingestellt und unter sterilen Bedingungen in Ampullen eingefüllt. Die Verdünnung hängt dabei von der ermittelten biologischen Aktivität, die in "Biologischen Einheiten" ausgedrückt und auf den Ampullen vermerkt wird, und dem beabsichtigten Verwendungszweck des damit hergestellten Präparates ab. Die in der beschriebenen Weise erhaltenen standardisierten Präparate enthalten genau definierte Mengen an immunologisch aktiven galaktosidspezifischen Mistellektinen und erlauben deren exakte Dosierung, so dass sowohl eine Schädigung des Patienten durch eine ungewollte Ueberdosierung wie auch eine durch eine zu niedrige Dosierung verursachte Unwirksamkeit auszuschliessen sind.

Die zur Herstellung der Mistellektin-Konzentrate der eingangs erwähnten Art durchzuführenden Fraktionierungsstufen werden zweckmässigerweise unmittelbar aufeinanderfolgend durchgeführt, wobei ebenso wie bei der nachfolgenden Herstellung der standardisierten Präparate auf die strikte Einhaltung steriler Bedingungen zu achten ist.

Stabilisierung der verdünnten bzw. gepufferten Lösung

Zur Erhaltung der galaktosidspezifischen Mistellektinaktivität wird ein Stabilisator im Bereich von 0,1 mg/ml bis 10 mg/ml, vorzugsweise 5 mg/ml, zugesetzt.

Hierfür geeignete Stabilisatoren sind Alkanole speziell Polyole und deren Derivate, wie z.B. Kohlenhydrate wie Glucose, Saccharose, Cyclodextrine, derivatisierte Cyclodextrine, Pentaerythrit, Mannit, Sorbit und Polyhydroxyfettsäuren wie Polymilchsäure und Polymilchsäureester, als auch andere hydrophile Polymere wie Polyvinylpyrrolidon, Polyvinylalkohol, als auch Aminsoäuren oder Proteine wie Glycin oder Albumin spez. Humanserumalbumin.

BESTIMMUNG DER BIOLOGISCHEN BZW. IMMUNOLOGISCHEN AKTIVITÄT

Untersuchungen in vitro

Materialien und Methoden

Für die Untersuchung wurden aus Mistelpflanzen gewonnene Extrakte verwendet, aus denen Mistellektine isoliert wurden. Der Gehalt an zuckerbindenden Lektinen wurde mittels eines Enzymtestes - Enzym-Linked-Lektin-Assay [ELLA] -, wie früher von Hajto, T., Hostanska, K., Gabius, H.-J.[**Cancer Res., 49** (1989) 4803 bis 4808] beschrieben, bestimmt. Für diese Untersuchung wurden ausserdem polyklonale Antikörper gegen Periodat-oxydiertes, mit Formaldehyd behandeltes ML I und Asialofetuin (Typ I) (von Sigma, St. Louis, USA) verwendet.

Bestimmung der Lebensfähigkeit der Zellen

Menschliche periphere mononukleäre Blutzellen (PBMC) wurden von verschiedenen Spendern mittels Gradientenzentrifugation nach Ficoll isoliert. Nach dem Waschen wurden die PMBC in serum-freiem Hybridom-Medium mit niedrigem Proteingehalt (von GIBCO BRL AG, Basel, Schweiz), welches mit L-Glutamin (2 mM) und 50 µg/ml Gentamycin ergänzt worden war, suspendiert. Die Zellen wurden in flachbodigen Mikro-Titerplatten (96 Vertiefungen) in einer Konzentration von $1 \times 10^6$ Zellen pro Vertiefung in Gegenwart oder Abwesenheit verschiedener Agentien, wie nachfolgend bei der Diskussion der Ergebnisse angegeben, während 24 Stunden bei 37°C in einer feuchten Atmosphäre mit einem Gehalt von 5% $CO_2$ inkubiert. Unabhängig davon wurde die Lebensfähigkeit der Zellen mittels des Trypan-Blau-Ausschlusstests sowie der Aufnahme von Fluoresceinacetat und dessen nachfolgender Hydrolyse zu Fluorescein bestimmt.

Bestimmung der Zytotoxizität

Die Untersuchung der Zytotoxizität natürlicher Killerzellen, nachfolgend als NK-Zytotoxizität bezeichnet, wurde wie im Detail von Hajto, T. Hostanska, K., Gabius, H.-J. [**Cancer Res., 49** (l.c.)] und Hajto, T., Hostanka, K. [**Clin. Trials J., 22** (1985) 514 bis 520] beschrieben, durchgeführt. Dabei wurden mononukleäre Zellen mittels des Ficoll-Hypaque-Gradienten isoliert. Eine vorgegebene Menge NK-sensitiver $K_{562}$-Zielzellen, nämlich $2,5 \times 10^3$ Zellen, wurden zu Effektorzellen in unterschiedlichen Konzentrationen hinzugefügt, um im Endeffekt Mischungsverhältnisse von Effektorzellen zu Zielzellen von 100:1, 50:1, 25:1 und 10:1 in einem Endvolumen von 200 µl pro Vertiefung zu erhalten. Unmittelbar nach der Herstellung der Zellsuspension wurden in die Vertiefungen 2 µCi [Methyl-$^3$H]-thymidin (spez. Aktivität: 25 Ci/mMol) eingebracht. Nach einer Inkubationsdauer von 18 bis 20 Stunden wurden die Zellen geerntet und die [$^3$H]-Thymidin-Aufnahme mittels eine Szintillationszählers quantitativ bestimmt. Die spontane Aufnahme der markierten Substanz stand in Beziehung zur Aktivität der Zielzellen. Der Prozentsatz der spezifischen Hemmung (Pi), ausgedrückt durch den cytotoxischen Index der Effektorzellen wurde nach der folgenden Formel berechnet:

$$Pi = (1 - \frac{\text{Testaufnahme}}{\text{Spontane Aufnahme}}) \times 100$$

Der [$^3$H]-Thymidineinbau in die Effektorzellen wurde ebenfalls in ähnlicher Weise bestimmt. Da der hierbei erhaltene Wert jedoch vernachlässigbar niedrig war, konnte dieser Faktor ausser Betracht gelassen werden. Die Anzahl von Effektorzellen, die eine spezifische Hemmung von 33% (Pi - 33) ergaben, wurde mittels eines halblogarithmischen Massstabes berechnet. Diese Pi 33-Werte wurden als wertvolles Hilfsmittel für den Vergleich verschiedener experimenteller Resultate verwendet und als spezifische Zytotoxizität ausgedrückt. Darüberhinaus wurden morphologische Untersuchungen in den für die NK-Untersuchungen isolierten mononukleären Zellpopulationen durchgeführt. Für andere Zellen als Lymphozyten wurde ein Korrekturfaktor ermittelt. Für In-vivo-Untersuchungen der NK-Aktivität wurde eine Gruppe von 12 weissen New Zealand-Kaninchen (NZW) eingesetzt.

Eine Kontamination durch Endotoxine wurde rigoros ausgeschlossen, wie ausführlich von Hajto, T., Hostanska, K., Gabius, H.-J. [**Cancer Res., 49** (l.c.)] beschrieben. Alle Daten wurden unter Anwendung einer statistischen Methode, und zwar mittels des t-Tests nach Student ermittelt.

Lebensfähigkeit von menschlischen peripheren mononukleären Blutzellen in Gegenwart von Mistellektin I (ML I)

Zur Erläuterung der zytotoxischen und/oder zytostatischen Wirkung von ML I wurde die Lebensfähigkeit von PBMC in vitro in Anwesenheit von verschiedenen Konzentrationen des Lektins bestimmt. Die Lebensfähigkeit der Zellen wurde durch Lektin-Konzentrationen $\geq$ 10 ng/ml signifikant ($p<0,05$) beeinträchtigt, vgl.: Hajto, T., Hostanska, K., Frei, K., Rordorf C., Gabius, H.-J. [**Cancer Res., 50** (l.c.)]. Bei Anwendungen kleinerer, nicht-toxischer Gaben zeigte ML I eine leicht mitogene Wirkung, was mit den Erkenntnissen anderer Autoren [Franz, H., **Oncology**, **43,** Supplement 1, (1986) 23 bis 24; Metzner, G., Franz, H., Kindt, A., Fahlbusch, B., Süss, J., **Immunbiol., 169** (1985) 461 bis 471; Luther, P., Theise, H., Chatterjee, B., Karduck, D., Uhlenbruck, G. **Int. J. Biochem., 11** (1980) 429 bis 435] im Einklang steht. Die optimale Immunantwort hinsichtlich der Lymphozytenvermehrung mit einem Stimulations-Index von 1,7 wurde bei einer Lektinkonzentration von 0,01 ng/ml gefunden. Sowohl die zytotoxische wie auch die mitogene Wirkung wurden bei Anwesenheit des spezifischen Zuckers (D-Galaktose) durch Verhinderung der Lektin-Bindung aufgehoben. Daraus folgt die Bedeutung der Wechselwirkungen zwischen Proteinen und Kohlenwasserstoffen im Verlauf der zellulären Reaktionen von ML I.

Wirkungen von Mistellektinen auf NK-sensitive Zielzellen [$K_{562}$] und auf die NK-Zytotoxizität in vitro

$K_{562}$-Zellen sind menschliche myeloische Leukämiezellen, die gegenüber der Zytotoxizität von NK-Zellen in hohem Masse empfindlich sind und in weitem Rahmen für die Bestimmung der NK-Aktivität von PMBC verwendet werden. In der Kultur von $K_{562}$-Zellen zeigten alle drei Arten von Mistellektinen eine verhältnismässig starke zytotoxische Wirksamkeit, [Hajto, T., Hostanska, K.,: **Interlec Meeting,** Berlin 1991). Die oben erwähnte Ueberempfindlichkeit gegenüber Lektinen wurde auch in Kulturen anderer Leukämiezellen (MOLT 4) beobachtet [Ribereau-Gayon, G., Jung, M.L., DiScala, D., Beck, J-P. **Oncology, 43,** Supplement 1, (1986) 35 bis 41]. Im Gegensatz zu diesen Tumor-Zellinien zeigte ML I in Kulturen von gesunden menschlichen PMBC zytotoxische Wirkungen, die um drei bis vier Grössenordnungen kleiner waren als die in Kulturen von Leukämiezellen gefundenen. Folglich ist es nicht überraschend, dass Mistellektine, wenn sie in niedrigen, für die Lymphozyten nicht-toxischen und leicht mitogenen, für die Zielzellen jedoch toxischen Konzentrationen inkubiert werden, die zytotoxische Aktivität von NK-Zellen gegenüber $K_{562}$-Zellen erhöhen können, [Hajto, T., Hostanska, K.: **Interlec Meeting,** Berlin 1991]. Es war daher von besonderem Interesse, zu untersuchen, ob es die In-vivo-Ueberwachung von NK-Zellen ermöglicht, für das Immunsystem nicht-toxische und für die klinische Anwendung geeignete optimale Lektin-Dosierungen, die in Form von Mistelextrakten an Tumorpatienten verabreicht werden können, zu finden.

Untersuchungen in vivo

Wirkung von Mistellektin I auf die zytotoxische Aktivität von NK-Zellen in vivo

Um die möglicherweise vorhandene Bedeutung des NK-Assays für die nützliche Anwendung von Mistellektinen in der Klinik zu bestätigen, wurde als nächster Schritt die Wirkung unterschiedlicher Dosierungen von ML I bei einer Gruppe von NZW-Kaninchen untersucht. Die maximale Zunahme der NK-Aktivität wurde zwischen 24 und 72 Stunden nach einer einzelnen intravenösen Injektion beobachtet und zeigte, dass die von der angewandten Dosierung abhängigen Immunantworten in vivo, den in vitro gefundenen ähnlich waren [Hajto, T., Hostanska, K: **Interlec Meeting,** Berlin 1991], wie aus der Figur, welche die Abhängigkeit der Zunahme der NK-Aktivität im peripheren Blut von NZW-Kaninchen zwischen der 24. und 72. Stunde nach einer einmaligen intravenösen Injektion von der verabreichten Dosierung zeigt, ersichtlich ist. Daraus geht hervor, dass die Herabsetzung der für das Immunsystem toxischen Lektin-Dosierungen in einem Auftreten einer NK-stimulierenden Wirkung, mit einem Optimum bei 0,8 ng/kg bei Kaninchen resultiert, die um vier bis fünf Grössenordnungen niedriger ist als der $LD_{50}$-Wert von ML I. Darüberhinaus wurde eine ähnlich optimale Dosierung dieser Komponente des Extraktes bei Patienten [1 ng/kg] gefunden, die mit einem, aufgrund des Gehaltes an zuckerbindendem Lektin standardisierten Mistelextrakt behandelt worden waren: Hajto, T., Hostanska, K., Gabius, G.H. [**Therapeutikon, 4** (1990) 136 bis 145]. Sowohl die NK-Aktivität wie auch die Häufigkeit der im Blut zirkulierenden LGL Zellen wurden durch ML I stimuliert: Hajto, T., Hostanska, K., Gabius, H.-J. [**Cancer Res., 49** (l.c.)].

Vergleich der Wirkung von Mistellektinen (ML I und ML II) auf die NK-Zytotoxizität in vivo

Es wurde in fundierter Weise erwiesen, dass die drei, in Mistelextrakten enthaltenen, Arten von Lektinen (ML I, ML II und ML III) untereinander in Wechselwirkung stehen. Diese Tatsache muss bei deren Bestimmung mittels ELISA oder ELLA in Betracht gezogen werden: Ziska, P., Franz, H. [**Lectins, Vol. IV** (1985) 473 bis 480]. Trotz der Tatsache, dass der Anteil von ML II und ML III in Mistelpflanzen und einer grossen Zahl von deren Extrakten nur etwa 15% der Gesamtmenge von ML I beträgt, vgl. Ziska, P., Franz, H. [**Lectins, Vol. IV** (l.c.)] können gewisse Aenderungen ihrer Mischungsverhältnisse als Folge des Herstellungsprozesses bei verschiedenen Extrakten (ISCADOR) nicht ausgeschlossen werden. Folglich ist für eine therapeutische Anwendung dieser Extrakte eine Untersuchung der immunmodulierenden Wirksamkeit von ML II und ML III ebenfalls notwendig. ML III war bei der Verabreichung in der gleichen Dosierung, wie sie sich für ML I als optimal erwiesen hatte, nicht in der Lage die NK-Zytotoxizität in Kaninchenblut signifikant zu stimulieren (p>0,05). Im Gegensatz zu ML III zeigte ML II signifikante (p<0,025) jedoch quantitativ weniger ausgeprägte Wirkungen auf die NK-Aktivität bei Verabreichung in geeigneten Dosierungen im Vergleich zu ML I: Hajto, T., Hostanska, K., Gabius, H.-J., [**Cancer Res., 49** (l.c.)].

### Wirkung von Mistellektin I auf das Netzwerk von Zytokinen und Lymphokinen

Durch Ueberwachung von NK-Zellen wurde eine optimale Anwendung von Mistellektin gefunden, durch die zahlreiche Parameter des Abwehrsystems des Empfängers stimuliert werden können [Hajto, T., Hostanska, K., Gabius, H.-J. [**Cancer Res.,** 49 (l.c.) und Hajto, T., Hostanska, K., Gabius, G.H., **Therapeutikon 4,** (l.c.)] und die auf die mögliche Rolle der Zytokine schliessen lassen. Diese Annahme konnte in einer früheren Studie verifiziert werden: Hajto, T., Hostanska, K., Frei, K., Rordorf, C., Gabius, H.-J. [**Cancer Res., 50** (l.c.)]. Bei Herabsetzung der zytotoxischen Konzentration von ML I in einer Kultur von menschlichen PBMC auf einen Bereich, der für Lymphozyten nicht toxisch ist, wurde eine vermehrte Sekretion von Tumor-Nekrose-Faktor-alpha (TNF-alpha), Interleukin-1 (IL-1) und Interleukin-6 (IL-6) beobachtet. Diese durch Lektin vermehrte Sekretion von Zytokinen hängt von der spezifischen Bindung des Lektins an zelluläre Glucokonjugate ab. Darüberhinaus wurden die Serumspiegel von TNF-alpha und IL-6 bei acht Krebspatienten nach der Injektion eines Extraktes mit einem optimalen Lektingehalt (1 ng/kg) erhöht.

Diese, das Zytokin-Netzwerk betreffenden Untersuchungen sind bisher nicht zu Ende geführt worden, es ist jedoch sehr wahrscheinlich, dass TNF-alpha, IL-1 und IL-6 eine entscheidende Rolle bei der Vermittlung von Lektin-induzierter Verstärkung verschiedener Abwehrmechanismen des Empfängers spielen. Eine Lektin-induzierte NK-Vermehrung kann auch der vermehrten Freisetzung von IL-1, IL-6 und TNF-alpha zugeordnet werden, welche in der Lage ist, die Wechselwirkung zwischen IL-2-Rezeptoren auf NK-Zellen und IL-2 (Verstärkung der CD25-Ausprägung und IL-2-Synthes) zu verstärken. Weitere Untersuchungen zur Klärung dieser Fragen sind im Gange.

### Zusammenhang zwischen durch Mistellektine vermittelter immunmodulierender Wirkung und Antitumor-Aktivität

Nach der Bestimmung der optimalen immunmodulierenden Dosis von Mistellektin erhob sich die Frage, ob das Lektin eine Antitumor-Wirkung hervorrufen kann, wenn es für eine Langzeitanwendung eingesetzt wird. Im Laufe von Untersuchungen mit unterschiedlichen Dosierungen von ML I zeigte sich, dass nur die optimale Dosierung (1 ng/kg) eine Verringerung des Tumorwachstums bei nackten Mäusen mit einer Fremdtransplantation von menschlichem Tumor [Steinberg et al., Universität Essen (BRD), unveröffentlichte Daten] bewirkte. In ähnlicher Weise waren Mistelextrakte, die aufgrund des Gehaltes an Zucker-bindendem Lektin standardisiert und entsprechend einem optimierten Dosierungsschema verabreicht worden waren, in der Lage, bei Patienten mit Tumoren in fortgeschrittenem Krankheitsstadium eine Antitumor-Aktivität zu induzieren [Hajto, T., Hostanska, K., Fornalski, M., Kirsch, A. **Dtsch. Zeitschr. Onkol., 23** (1991) 1 bis 6]. Darüberhinaus zeigten Untersuchungen an Tiermodellen eine Schutzwirkung von ML I auf das NK-System bei seiner Verabreichung in Kombination mit einer Strahlentherapie, wie unveröffentlichte Daten, die in Einklang mit klinischen Beobachtungen sind, zeigten: Hajto, T., Hostanka, K., Gabius G.H. [**Therapeutikon, 4** (l.c.)].

### ERGEBNISSE UND DARAUS ZU ZIEHENDE SCHLUSSFOLGERUNGEN

Die vorstehenden Ausführungen, die sich auf im Laufe der beschriebenen Untersuchungen erhaltene Ergebnisse stützen, machen deutlich, dass eine Ueberwachung des NK-Systems, hinsichtlich der zytotoxischen Aktivät und/oder der Häufigkeit der im Blut zirkulierenden NK-Zellen, es ermöglichen kann, optimale Dosierung für hoch-toxische Mistellektine, wie sie in Mistelextrakten enthalten sind und die eine Stimulierung der Abwehrmechanismen eines Empfängers bewirken können, anzugeben.

Die genannten Ergebnisse lassen ausserdem mit an Sicherheit grenzender Wahrscheinlichkeit vermuten, dass für die Antitumor-Aktivität der Mistellektine sowohl deren zytotoxische Aktivität wie auch deren immunmodulierende Wirksamkeit notwendig sind.

Die erhaltenen Ergebnisse lassen auch erkennen, daß die Verabreichung von standardisierten, stabilisierten Mistellektin-Präparaten mit aufgrund ihrer biologischen Aktivität genau definiertem Gehalt an biologisch aktiven Mistellektinen nach einem optimierten Dosierungsplan eine Verwendung in der Tumor-Therapie und zur Bekämpfung anderer Krankheiten, z.B. durch Erhöhung der natürlichen Immunresistenz, empfehlenswert erscheinen läßt.

## Patentansprüche

1. Lektinkonzentrate aus Mistelextrakten und entsprechende standardisierte Mistellektinpräparate mit einem Gehalt von 50 ng/ml bis 350 ng/ml an immunologisch aktiven, galaktosidspezifischen, in Abhängigkeit von der ermittelten biologischen Aktivität verdünnten Mistellektinen, bestimmt als Mistellektin-I-Standard, wobei sich die Lektinmenge nur auf die biologisch aktiven Lektine bezieht.

2. Präparate nach Anspruch 1 in Form einer Lösung mit einem pH-Wert von 7,0 bis 7,5.

3. Präparate nach den Ansprüchen 1 bis 2, bei denen zur Erhaltung der Lektinaktivität Stabilisatoren zugesetzt sind.

4. Verfahren zur Herstellung von Mistellektinkonzentraten nach den Ansprüchen 1 bis 3 aus wäßrigen Mistelextrakten mittels einer fraktionierten Filtration, **dadurch gekennzeichnet, daß** man in einer ersten Fraktionierungsstufe aus dem unfermentierten, wäßrigen Mistelextrakt durch Ultrafiltration ein Konzentrat mit Substanzen mit Molekularmassen von 20 kDa bis 100 kDa gewinnt, in einer zweiten Fraktionierungsstufe einer Sterilfiltration unterwirft, die biologische Aktivität des Konzentrats bestimmt, die Konzentration der immunologisch aktiven Mistellektine bestimmt, das Konzentrat entsprechend der ermittelten biologischen Aktivität verdünnt und gegebenenfalls stabilisiert, den für die therapeutische Verwendung notwendigen Lektingehalt einstellt, portioniert, und die Dosiseinheiten in sterile und pyrogenfreie Ampullen einschließt.

5. Verfahren nach Anspruch 4, **dadurch gekennzeichnet, daß** man in der ersten Fraktionierungsstufe zwei Ultrafiltrationen durchführt, wobei man für die erste Ultrafiltration ein Polysulfonfilter mit einer nominalen Molekulargewichttrenngrenze (NMGT) von 100.000 verwendet, das Filtrat zweimal mit einer Pufferlösung wäscht und für die zweite Ultrafiltration ein Celluloseacetatfilter mit 20000 NMGT verwendet.

6. Verfahren nach Anspruch 4 oder 5, **dadurch gekennzeichnet, daß** man in der zweiten Fraktionierungsstufe zwei Sterilfiltrationen durchführt.

7. Verfahren nach einem der Ansprüche 4 bis 6 **dadurch gekennzeichnet, daß** man im Bereich von pH 7,0 bis 7,5 arbeitet.

8. Verfahren nach einem der Ansprüche 4 bis 7, **dadurch gekennzeichnet, daß** man die erste und zweite Fraktionierungsstufe unmittelbar aufeinanderfolgend innerhalb von 2 Stunden durchführt.

9. Verfahren nach den Ansprüchen 4 und 8, **dadurch gekennzeichnet, daß** man als Stabilisatoren Alkanole, speziell Polyole und deren Derivate wie Kohlenhydrate, z.B. Glucose, Saccharose, Cyclodextrine, derivatisierte Cyclodextrine, Pentaerythrit, Mannit, Sorbit und Polyhydroxyfettsäuren wie Polymilchsäure und Polymilchsäureester, als auch andere hydrophile Polymere wie Polyvinylpyrrolidon, Polyvinylalkohol, als auch Aminosäuren wie Glycin verwendet.

10. Verfahren nach den Ansprüchen 4, 8 und 9, **dadurch gekennzeichnet, daß** man die Stabilisatoren in einer Menge im Bereich von 0,1 mg/ml bis 10 mg/ml, vorzugsweise 5 mg/ml der verdünnten oder gepufferten Lösung zusetzt.

11. Verfahren nach Anspruch 4, **dadurch gekennzeichnet, daß** man die Bestimmung der biologischen Aktivität mit Hilfe von drei unabhängigen Testmethoden in vitro durchführt, wobei man den Anteil an zuckerbindenden aktiven Lektinen unter Anwendung eines optimierten Enzym-Tests bestimmt, die Zellaktivität in einer Kultur von menschlichen periphporenmononuklearen Zellen (PMNC) mittels Trypanblau und Fluoresceindiacetat, welches zu Fluorescein hydrolysiert wird, ermittelt und die Aufnahme von [$^3$H]-Thymidin bei lektinempfindlichen Leukämiezellen [$K_{562}$] bestimmt.

12. Verfahren'nach Anspruch 11, **dadurch gekennzeichnet, daß** man die Standardisierung der erhaltenen biologisch aktiven galaktosidspezifischen Mistellektinfraktion enthaltenden Präparate anhand der Werte aus der Bestimmung der biologischen Aktivität vornimmt.

13. Standardisierte stabilisierte Mistellektinpräparate mit definiertem Gehalt an immunologisch aktiven galaktosidspezifischen Mistellektinen definierter biologischer Aktivität, erhältlich nach einem Verfahren nach einem der Ansprüche 4 bis 12.

14. Arzneimittel, enthaltend standardisierte stabilisierte Mistellektine nach den Ansprüchen 1 bis 3 oder 13.

15. Verwendung der Mistellektine nach den Ansprüchen 1 bis 3 oder 15 zur Herstellung eines Arzneimittels zur Erhöhung der natürlichen Immunresistenz bei Menschen und Säugetieren und/oder für die Tumortherapie.

**Claims**

1. Lectin concentrates of mistletoe extracts and corresponding standardized mistletoe lectin preparations having a content of from 50 ng/ml to 350 ng/ml of immunologically active, galactoside-specific mistletoe lectins diluted according to the detected biological activity, determined as mistletoe lectin I standard, wherein the amount of lectins relates to the biologically active lectins only.

2. The preparations according to claim 1 in the form of a solution having a pH value of from 7.0 to 7.5.

3. The preparations according to claims 1 to 2, which preparations are added with stabilizers to preserve the lectin activity.

4. A process for the production of mistletoe lectin concentrates according to claims 1 to 3 from aqueous mistletoe extracts using fractionated filtration, **characterized in that** in a first fractionating stage, a concentrate including substances with molecular masses of from 20 kDa to 100 kDa is obtained from the unfermented, aqueous mistletoe extract by ultrafiltration, and in a second fractionating stage, said concentrate is subjected to a sterile filtration, the biological activity of the concentrate is determined, the concentration of immunologically active mistletoe lectins is determined, the concentrate is diluted according to the detected biological activity and optionally stabilized, the lectin content required for therapeutic use is adjusted, portioned, and the dosage units are sealed in sterile and pyrogen-free ampoules.

5. The process according to claim 4, **characterized in that** two ultrafiltrations are performed in the first fractionating stage, wherein a polysulfone filter having a nominal molecular weight cut-off (NMWC) of 100,000 is used in the first ultrafiltration, the filtrate is washed twice with a buffer solution, and a cellulose acetate filter having an NMWC of 20,000 is used in the second ultrafiltration.

6. The process according to claim 4 or 5, **characterized in that** two sterile filtrations are performed in the second fractionating stage.

7. The process according to any of claims 4 to 6, **characterized in that** operations are performed at a pH ranging from 7.0 to 7.5.

8. The process according to any of claims 4 to 7, **characterized in that** the first and second fractionating stages are carried out in immediate succession within 2 hours.

9. The process according to claims 4 and 8, **characterized in that** alkanols, particularly polyols and derivatives thereof, such as carbohydrates, e.g. glucose, saccharose, cyclodextrins, derivatized cyclodextrins, pentaerythritol, mannitol, sorbitol, and polyhydroxyfatty acids such as polylactic acid and polylactic acid esters, as well as other hydrophilic polymers such as polyvinylpyrrolidone, poly(vinyl alcohol), as well as amino acids such as glycine are used as stabilizers.

10. The process according to claims 4, 8 and 9, **characterized in that** the stabilizers are added to the diluted or buffered solution in an amount ranging from 0.1 mg/ml to 10 mg/ml, preferably 5 mg/ml.

11. The process according to claim 4, **characterized in that** the biological activity determination is performed *in vitro* using three independent testing methods, wherein the percentage of sugar-binding active lectins is determined using an optimized enzyme test, the cell activity in a culture of human peripheral pore mononuclear cells (PMNC) is detected using trypan blue and fluorescein diacetate which is hydrolyzed to yield fluorescein, and the uptake of $[^3H]$-thymidine in lectin-sensitive leukemia cells $[K_{562}]$ is determined.

12. The process according to claim 11, **characterized in that** the standardization of the preparations obtained, containing the biologically active galactoside-specific mistletoe lectin fraction, is effected with reference to the values from the biological activity determination.

13. Standardized stabilized mistletoe lectin preparations having a well-defined content of immunologically active, galactoside-specific mistletoe lectins of defined biological activity, which preparations are obtained using the process according to any of claims 4 to 12.

14. Drugs, containing the standardized stabilized mistletoe lectins according to claims 1 to 3 or 13.

15. Use of the mistletoe lectins according to claims 1 to 3 or 13 in the production of a drug used to increase the natural immune resistance in humans and mammals and/or in tumor therapy.

**Revendications**

1. Concentrés des lectines, obtenus à partir d'extraits de gui et préparations des lectines de gui correspondantes normalisées ayant une teneur de 50 ng/ml à 350 ng/ml en lectines de gui immunologiquement actives, spécifiques au galactoside, diluées en fonction de l'activité biologique recherchée, déterminées comme standard I des lectines de gui, dans lesquelles la quantité des lectines se rapporte uniquement aux lectines biologiquement actives.

2. Préparations d'après la revendication 1 sous forme d'une solution ayant un pH de 7,0 à 7,5.

3. Préparations d'après les revendications 1 à 2, dans lesquelles des stabilisants sont ajoutés pour conserver le potentiel actif de la lectine.

4. Procédé de fabrication de concentrés des lectines de gui d'après les revendications 1 à 3, obtenus à partir d'extraits aqueux de gui par filtration fractionnée, **caractérisé en ce qu'**on obtient en une première étape de fractionnement à partir d'un extrait aqueux de gui non fermenté, par ultrafiltration, un concentré comportant des substances ayant une masse molaire de 20 kDa à 100 kDa, on soumet le concentré à une filtration stérilisante lors d'une deuxième étape de fractionnement, on détermine l'activité biologique du concentré, on détermine la concentration des lectines de gui immunologiquement actives, on dilue le concentré selon l'activité biologique recherchée et on le stabilise le cas échéant, on dose la teneur en lectines nécessaire pour une utilisation thérapeutique, on la divise, et on introduit les doses dans des ampoules stériles et exemptes de substances pyrogènes.

5. Procédé d'après la revendication 4, **caractérisé en ce qu'**on effectue deux ultrafiltrations dans la première étape de fractionnement, dans lesquelles on utilise pour la première ultrafiltration un filtre en polysulfone ayant une limite nominale de séparation de masse molaire (NMGT) de 100.000, on rince le filtrat deux fois avec une solution tampon, et on utilise pour la deuxième ultrafiltration un filtre en acétate de cellulose ayant une NMGT de 20.000.

6. Procédé d'après la revendication 4 ou 5, **caractérisé en ce qu'**on effectue dans la deuxième étape de fractionnement deux filtrations stérilisantes.

7. Procédé d'après une des revendications de 4 à 6, **caractérisé en ce qu'**on travaille dans une gamme de pH de 7,0 à 7,5.

8. Procédé d'après une des revendications de 4 à 7, **caractérisé en ce que** l'on effectue la première et la deuxième étape de fractionnement aussitôt l'une après l'autre dans un délai maximal de deux heures.

9. Procédé d'après les revendications 4 et 8, **caractérisé en ce qu'**on utilise, comme stabilisants, des alcanols, spécialement des polyols et leurs dérivés comme dès hydrates de carbone, par ex. du glucose, de la saccharose, des cyclodextrines, des cyclodextrines modifiées, du pentaérythritol, du mannitol, du sorbitol et des acides gras polyhydroxylés, comme l'acide polylactique et les esters d'acide polylactique, ainsi que d'autres polymères hydrophiles comme du polyvinylpyrrolidone, de l'alcool polyvinylique, ainsi que des aminoacides comme la glycine.

10. Procédé d'après les revendications 4, 8 et 9, **caractérisé en ce qu'**on ajoute les stabilisants en une quantité représentant de 0,1 mg/ml à 10 mg/ml, de préférence 5 mg/ml de la solution diluée ou tamponnée.

11. Procédé d'après la revendication 4, **caractérisé en ce qu'**on effectue la détermination de l'activité biologique in vitro au moyen de trois méthodes d'essai indépendantes, dans lesquelles on détermine la proportion des lectines active's formant une liaison avec le sucre en utilisant un dosage enzymatique optimisé, on mesure l'activité cellulaire dans une culture de cellules mononucléaires à pores périphériques (PMNC) humaines au moyen de bleu trypan et de diacétate de fluorescéine hydrolysé en fluorescéine, et on détermine l'absorption de la [$^3$H]-thymidine dans des cellules leucémiques [$K_{562}$] sensibles aux lectines.

12. Procédé d'après la revendication 11, **caractérisé en ce qu'**on effectue la normalisation des préparations obtenues, contenant une fraction des lectines de gui spécifique au galactoside biologiquement active, en s'appuyant sur les valeurs obtenues en déterminant l'activité biologique.

13. Préparations des lectines de gui stabilisées et normalisées ayant une teneur définie en lectines de gui spécifiques au galactoside immunologiquement actives possédant une activité biologique définie obtenues selon un procédé d'après une des revendications de 4 à 12.

14. Médicament contenant des lectines de gui normalisées et stabilisées d'après les revendications 1 à 3 ou 13.

15. Utilisation des lectines de gui d'après les revendications 1 à 3 ou 13 pour la fabrication d'un médicament visant à augmenter la résistance immunitaire naturelle chez l'homme ou les mammifères et/ou pour soigner les tumeurs.

MAXIMALE ZUNAHME DER ZYTOTOXIZITÄT ± SE

20

15

10

5

0

100  50  10  1  0,8 0,5  0

VERABREICHTE DOSIS[ng/kg]